Europäisches Patentamt

**European Patent Office**

Office européen des brevets

(11) Veröffentlichungsnummer: **0 215 735**
**B1**

(12) **EUROPÄISCHE PATENTSCHRIFT**

(45) Veröffentlichungstag der Patentschrift: **13.06.90**

(21) Anmeldenummer: **86810383.9**

(22) Anmeldetag: **27.08.86**

(51) Int. Cl.⁵: **G 01 N 33/84, C 12 M 1/24,**
**C 12 Q 1/56, B 01 L 3/14**

(54) Veterinärmedizinischer Reagentiensatz für Schnelltest zur Bestimmung des Blutcalciumgehaltes.

(30) Priorität: **05.09.85 CH 3828/85**

(43) Veröffentlichungstag der Anmeldung:
**25.03.87 Patentblatt 87/13**

(45) Bekanntmachung des Hinweises auf die
Patenterteilung:
**13.06.90 Patentblatt 90/24**

(84) Benannte Vertragsstaaten:
**AT BE CH DE FR GB IT LI LU NL SE**

(56) Entgegenhaltungen:
**DE-A-2 533 052**
**FR-A-2 029 242**
**FR-A-2 245 947**
**FR-A-2 425 642**
**US-A-4 239 746**

(73) Patentinhaber: **Dr. Ernst Gräub AG**
**Rehhagstrasse 83**
**CH-3018 Bern (CH)**

(72) Erfinder: **Hugi, Franz Niklaus**
**Dr. E.Gräub AG Rehhagstrasse 83**
**CH-3018 Bern (CH)**
Erfinder: **Rüetschi, Donatus**
**Dr. E.Gräub AG Rehhagstrasse 83**
**CH-3018 Bern (CH)**

(74) Vertreter: **Fischer, Franz Josef et al**
**BOVARD AG Patentanwälte VSP**
**Optingenstrasse 16**
**CH-3000 Bern 25 (CH)**

Anmerkung: Innerhalb von neun Monaten nach der Bekanntmachung des Hinweises auf die.Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft einen Reagentiensatz für die veterinärmedizinische Diagnostik, nämlich für die semiquantitative Bestimmung de Blutcalciumgehaltes bei Tieren, insbesondere bei Kühen. Die Erfindung betrifft ebenfalls ein Verfahren zur Herstellung des erfindungsgemässen Reagentiensatzes und ein Verfahren zur Bestimmung des Blutcalciumgehaltes unter Verwendung des erfindungsgemässen Reagentiensatzes.

Beim Rind ist hypocalcaemisches Fetliegen eine häufig vorkommende Krankheit. Zur Diagnose dieser Krankheit ist es erforderlich, den Calciumgehalt des Serums mindestens semiquantitativ zu bestimmen. Es besteht deshalb ein Bedürfnis nach Diagnostika, durch welche sofort und auf einfache Weise schon im Stall der Blutcalciumgehalt von Tieren bestimmt werden kann.

J. Martig, H. Gerber und M. Berger in "Die Deutsche Tierärztliche Wochenschrift" 6, 132-135 (1974) beschreiben den Vergleich eines Stalltestes mit einer Labormethode zur Bestimmung der Calciumkonzentration im Blut von Kühen. Beim beschriebenen Stalltest wurde EDTA-Lösung verwendet. In diesem Test war eine mindestens 2 h lange Wartezeit erforderlich und die semiquantitative Bestimmung stimmte nur in 72 % der Fälle. Der in dieser Publikation erwähnte Stammtest war erstmals durch Mayer in "J. Americ. Vet. Med. Ass.", 146/8, 839-842, beschrieben. Dieses Verfahren wurde durch Sandholm et al. verbessert, indem anstelle der direkten Titration des Calciums mit EDTA dem Blut zuerst ein EDTA-Ueberchuss zugegeben wird und dieser dann mit Calciumionen in Gegenwart von Thromboplastin rücktitriert wird. Der Reaktionsprozes wird durch einen Zusatz von Thromboplastin beschleunigt, so dass eine Ablesung bereits nach 5 min erfolgen kann (M. Sandholm et al.,"Zbl. Vet. Med. A", 26, 411-416 (1979)). Ein auf diesem verbesserten Verfahren beruhender Ca-Test wurde von der Firma Orion SF-00101 Helsinki 10 in den Handel gebracht. Dieser Test hat jedoch den Nachteil, dass zu seiner Durchführung verschiedene Flaschen bzw. Röhrchen erforderlich sind. Dabei muss nämlich zuerst das Testblut in ein Röhrchen mit EDTA gegeben werden, wobei der Komplexbildner im Blut aufgelöst und dieses ungerinnbar wird,. zudem in ein verschliessbare Fläschchen, welches Calciumchlorid und Thromboplastin enthält, destilliertes Wasser (10 Tropfen) zur Auflösung gegeben wird, das Tetblut mit dem Komplexbildner ebenfalls in dieses Fläschchen gegeben wird, welche schliesslich verchlossen und bewegt wird. Gerinnt die Probe nach 5 min, ist der Test positiv, findet jedoch keine Gerinnung statt, ist er negativ. Für den praktischen Einsatz beim Tierarzt it dieses Verfahren für die Durchführung in Feld und Stall aufwendig. Es wurden weitere Versuche unternommen, dieses Verfahren zu vereinfachen. So beschreibt A. Linhart in "Untersuchungen über die Brauchbarkeit eines Schnelltests zur semiquantitativen Bestimmung des Blutcalciumgehaltes beim Rind", Vet. Med. Dissertation, München (1984) und in der entsprechenden Publikation von W. Klee, A. Linhart und D. Schillinger in der tirrärztlichen Umschau 40, 450-454 (1985) eine Vereinfachung der Methode von Sandholm. Dieses Verfahren ist wiederum ein direktes Verfahren, welches ohne zusätzliche Calciumionen durchgeführt wird. Dieser Test ist bezüglich der Handhabung etwas einfacher als der ursprüngliche Sandholm-Test, ist ihm jedoch bezüglich der Testergebnisse eher unterlegen.

Es ist demzufolge Aufgabe der vorliegenden Erfindung, Testreagentien in einer Fertigpackung vorzulegen, die eine einfache Durchführung des Verfahrens zur semiquantitativen Bestimmung des Blut-Calciumgehaltes ermöglichen.

Diese Aufgabe wurde erfindungsgemäss durch den im Patentanspruch 1 beschrieben Reagentiensatz in einem Teströhrchen und dem dazugehörenden Stopfen gelöst.

Grundsätzlich entspricht das Testverfahren dem durch Sandholm beschriebenen Verfahren. Die Verpackungsweise der Reagentien im Teströhrchen bzw. in dem dazugehörigen Stopfen ermöglicht es, dass der Test durch den Tierarzt unter den Bedingungen des Stalls schnell und sicher durchgeführt werden kann. Beispielsweise werden Fehler durch Umfüllen der Testlösung oder durch Verunreinigungen auf ein Minimum beschränkt. Der Stopfen des Teströhrchens ist vorzugsweise entsprechend der im Patentanspruch 2 beschriebenen Ausführungsform ausgebildet und besteht normalerweise aus Polyethylen. Das Röhrchen besteht in der Regel aus Kunststoff, wie polyethylen oder Polypropylen und weist vorzugsweise eine Kalibrierung auf, die dem Verbraucher angibt, welche Blutmenge zum Messen eines bestimmten Schwellenwertes, bezogen auf die im System enthaltenen Reagentien, erforderlich ist.

Die drei chemischen Reagentien, welche im Testsystem eingesetzt werden, sind:

Ein Komplexbildner zum Komplexieren der Calciumionen, beispielsweise Ethylendiamintetraessigsäure (EDTA), deren Salze, wie Natrium oder Kaliumsalz, Ethylenglykol-bis-(2-aminoethyl)-tetraessigsäure (EGTA) und ihre Salze, wie Natriumoder Kaliumsalz; ein Calciumsalz zur Rücktitration des überschüssigen Komplexbildners, z.B. Calciumchlorid, Calciumascorbat, Calciumborogluconat, Calciumbromid, Calciumbromolactobionat, Calciumcarbonat, Calciumcyclamat, Calcium-2-ethylbutanoat, Calciumfluorid, Calciumformiat, Calciumgluconat, Calciumglycerophosphat, Calciumhypophosphit, Calciumjodid, Calciumlactat, Calciumlaevulinat, Calciummethionat, Calciumnitrat, Calciumnitrit, Calciumpantothenat, Calciumpropionat, Calciumselenat, Calciumsuccinat, Calciumsulfat, Calciumsulfit, Calciumtartrat, Calciurnthioglycollat, und Calciumthiosulfat;

Thromboplastin als Koagulationsbechleuniger. Das Thromboplastin stammt in der Regel aus tierischem Hirngewebe, z.B. vom Pferd, Rind, Schwein, Schaf, Kaninchen oder von der Ziege.

Das Röhrchen enthält als Komplexbildner vorzugsweise 25 µmol EDTA und im Deckel sind vorzugsweise 13,3 µmol Calcium und 12 mg Thromboplastin enthalten.

Mit den obengenannten Reagentienmengen weist da Röhrchen Marken auf bei den Mengen 14,2 ml, 10 ml, und 7,7 ml, welche den Schwellenwerten des Serumcalciumgehaltes von 5, 7 und 9 mg% bzw. 1,25, 1,75 und 2,25 mmol/Liter entsprechen. Mit dieser Kalibrierung genügen für ein Röhrchen weniger als 20 ml und für die Füllung von drei Röhrchen bei den verschiedenen Marken 32 ml. Dabei muss beachtet werden, dass zur genauen Abmessung vorzugsweise eine neue Einmalspritze aus Kunststoff mit einer neuen Einmalkanüle mittleren Durchmessers verwendet werden sollte. Zur Vermeidung einer vorzeitigen Aktivierung des Gerinnungssystems sollte vermieden werden, dass das Blut mit Glas (Gefässe oder Spritzen) in Kontakt kommt. Zur Herstellung des Reagentiensatzes wird in der Regel entsprechend dem im Anspruchssatz beschriebenen Herstellungsverfahren vorgegangen.

Die semiquantitative Bestimmung erfolgt gemäss dem allgemeinen Verfahren, welches im Patentanspruch 11 beschrieben ist.

Bei Durchführung des Tests im Stall wird dem Tier z.B. mit einer sauberen trockenen Spritze mit Kanüle zügig Vollblut aus der Euter-oder Halsvene entnommen. Das Blut wird nun in einer Menge entsprechend dem gewünschten Schwellenwert in das geöffnete Röhrchen gegeben. Dabei muss beachtet werden, dass die im Röhrchen vorhandene Substanz vollständig im Röhrchen bleibt. Befindet sich die gewünschte Blutmenge im Röhrchen, wird der Stopfen aufgesetzt und das Röhrchen wird einige Male geschwenkt, um den Komplexbildner im Blut aufzulösen und die Komplexierung des Calciums zu bewirken, wobei bis zu diesem Zeitpunkt der Inhalt dieses Stopfens nicht mit dem Blut in Kontakt kommen darf. Erst in diesem Zeitpunkt wird der Inhalt des Stopfens dem Testblut zugänglich gemacht, wobei der Inhalt des Stopfens vollständig mit dem Blut in Kontakt gebracht und vermischt werden muss.

Vom Zeitpunkt der vollständigen Vermischung muss 5 min gewartet werden, bis das Testergebnis beurteilt werden kann. Dabei muss festgestellt werden, ob das Blut koaguliert oder nicht. Eine vollständige Koagulation ist vorhanden, wenn sich im Röhrchen ein Blutpropfen bildet, der auch beim Wenden am Boden haften bleibt. Dies bedeutet, dass der Blutcalciumspiegel über dem gewählten Schwellenwert entsprechend der eingesetzten Menge Blut liegt. Enthält das Blut gallertartige Klumpen, was durch langsames Ausleeren erkennbar ist, liegt eine teilweise Koagulation vor, was bedeutet, dass der Blutcalciumspiegel bei oder über dem gewählten Schwellenwert liegt. Bleibt das Blut dünnflüssig, liegt keine Koagulation vor, womit festgestellt wird, dass der Blutcalciumspiegel unter dem gewählten Schwellenwert liegt.

In Zweifelsfällen kann die Beurteilung der Koagulation nach 10 oder 15 min wiederholt werden. Ausnahmsweise kann in dieser Zeit noch eine Aenderung, d.h. eine Intensivierung der Koagulation oder eine verspätete Koagulation eintreten.

In unklaren Fällen wird empfohlen, den Inhalt des Röhrchens langsam zu entleeren, wobei Anzeichen einer teilweisen Gerinnung durch Klumpen oder Fäden bemerkt werden können. Abgesehen von ungenauer Durchführung des Testes, wie Pipettierfehler sind folgende, vom Untersucher unabhängige Fehler möglich:

1. Veränderter Hämatokritwert

Der Test ist auf physiologische Hämatokritwerte eingestellt. Wenn der Hämatokritwert der Probandin stark vom physiologischen Bereich abweicht, kann es zu Fehlinterpretationen kommen.

Eine stärkere Erhöhung des Hämatokritwertes kann vor allem bei festliegenden Kühen auftreten, die nicht trinken können oder wollen Untersuchungen haben gezeigt, dass der Test dann im schlimmsten Fall 1 mg% bzw. 0,25 mmol/l zu tief anzeigt.

2. Vorzeitige Blutgerinnung

Bei Verwendung unsauberer Spritzen oder Kanülen sowie bei Verzögerung während der Blutübertragung ins Röhrchen kann die physiologische Blutgerinnung einsetzen und zu falschpositiven Ergebnissen führen.

Die semiquantitative Bestimmung mittels des erfindungsgemässen Reagentiensatzes ist insbesondere erforderlich bei

atypischer Gebärparese zur Erleichterung der Differentialdiagnose, z.B. bei Kühen, deren Sensorium trotz Festliegen weniger oder gar nicht beeinträchtigt ist und bei denen Traumen nicht die Ursache des Festliegens sind (Downer Cows); solche Tiere sind sogar vielfach in der Lage, sich mit Hilfe der Vordergliedmassen kriechend fortzubewegen, kommen aber mit der Nachhand nicht hoch (Creeper Cows). Diese Fälle zeichnen sich meist durch einen besonders ausgeprägten und selbst nach Calciuminfusionen weiter anhaltenden und bald rezidivierendern Abfall des Serumphosphorgehaltes bei nur mässig erniedrigtem Calciumgehalt aus.

Verdacht auf Hypocalcaemie, z.B. bei Kühen, die zwar noch stehen, bei denen aber gewisse Anzeichen auf eine beginnende Hypocalcaemie hinweisen (schlafender Blick, atonischer Uterus, u.a.) und bei Kühen mit starkem Durchfall.

Festliegen unklarer Genese: Zur Differentialdiagnose von Festliegen verursacht durch Hypocalcaemie gegenüber Festliegen verursacht durch andere Ursachen, wie geburtsbedingte Verletzungen im Beckenbereich, puerperale Genitalinfektionen, akute Euterentzündungen, hochgradige alimentäre Intoxikationen usw.

Komatöser Gebärparese, da hier ein erhöhtes Risiko bei einer Calciuminfusion besteht.

Rezidivierender Gebärparese zur Ueberprüfung des Behandlungserfolges bzw. zur Beantwortung der Frage einer Nachbehandlung.

Die Erfindung wird anhand einem in den Zeichnungen dargestellten Ausführungsbeispiel näher erläutert.

Es zeigen:

Fig. 1 in perspektivischer Darstellung eine Ansicht des Teströhrchens mit aufgesetztem Stopfen;

Fig. 2 einen Längsschnitt des aufgestzten Stopfens in geschlossenem Zustand;

Fig. 3 eine Ansicht des Stopfens von oben; und

Fig. 4 einen Längsschnitt des Stopfens in geöffnetem Zustand.

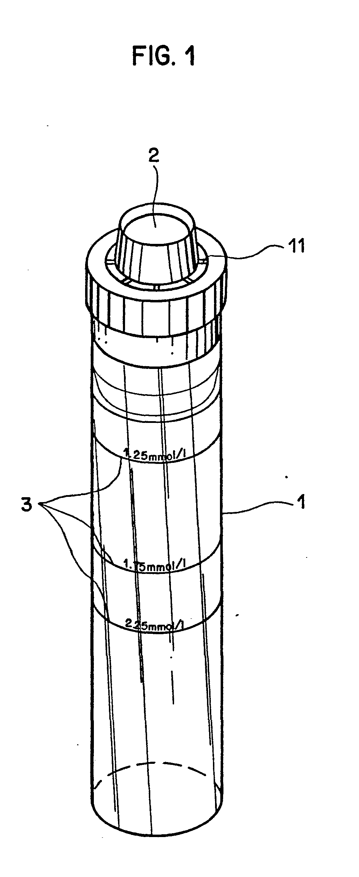

Das in Fig. 1 dargestellte Teströhrchen 1 mit dem aufgesetzten Stopfen enthält sämtliche Reagentien, welche für den Test erforderlich sind. Die Reagentien sind in trockenem, vorzugsweise gefriergetrocknetem Zustand und in einer genau bestimmten Menge vorhanden. Der Komplexbildner, vorzugsweise K2-EDTA, befindet sich am Boden des Teströhrchens 1 und das Thromboplastin und die Calciumverbindung befinden sich im verchlossen Innenraum des Stopfens 2.

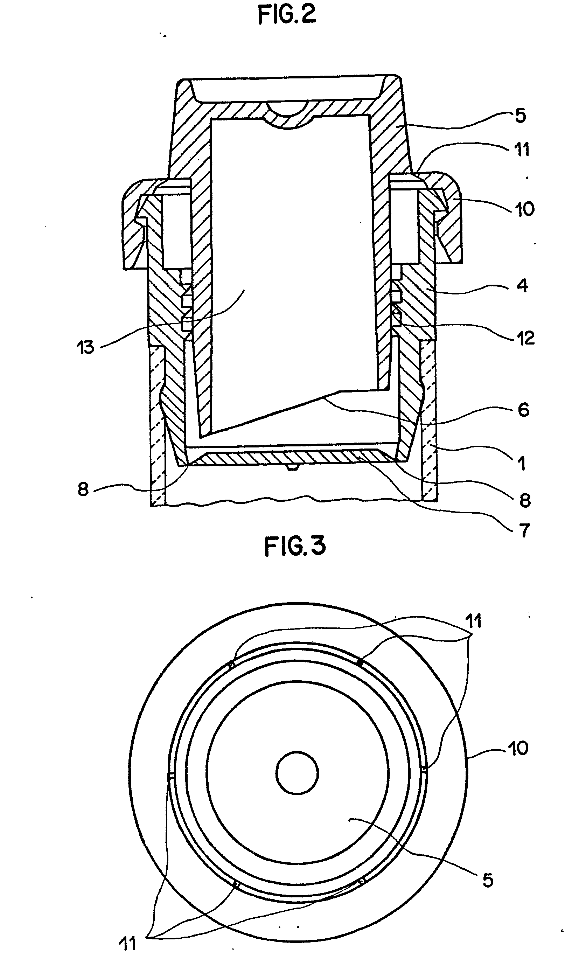

Die Einzelheiten einer bevorzugten Ausführungsform des Stopfens 2 sind in den Figuren 2-4 dargestellt. In Figur 2 ist ein Längsschnitt des Stopfens in geschlossenem Zustand dargestellt. Der Stopfen besteht im wesentlichen aus den beiden becherförmigen Teilen 4 und 5, welche den abgeschlossenen Raum 13 bilden, welcher zur Aufnahme des Thromboplastins und der Calciumverbindung dient. Der äussere becherförmige Teil weist Dichtungsrippen 12 auf, welche dafür sorgen, dass der Raum 13 dicht ist. Der äussere becherförmige Teil 4 dient ebenfalls zur Abdichtung gegenüber dem Teströhrchen 1. Der innere becherförmige Teil 5 ist beweglich, sodass nach Druck von oben der teilweise abgeschrägte Rand 6 den Bodenteil 7 deckelartig gegen das Innere des Röhrchens öffnet. Der Bodenteil 7 weist am Rand eine Reisslinie 8 auf, welche zur Erleichterung des Oeffnungsvorganges dient. Gegen ungewolltes Oeffnen dient der Sicherungsring 10, welcher den äusseren becherförmigen Teil 4 überlappt und bei kräftigem Druck von oben auf den inneren becherförmigen Teil 5 abgetrennt wird. Im ursprünglichen Zustand ist der Ring 10 durch mehrere Sollreiss-Stellen 11 mit dem inneren becherförmigen Teil verbunden.

Fig. 3 zeigt eine Oberansicht des Stopfens. Im Zentrum ist der obere Teil des inneren becherförmigen Teils 5 ersichtlich, der durch die Reissstellen 11 mit dem Sicherungsring 10 verbunden ist.

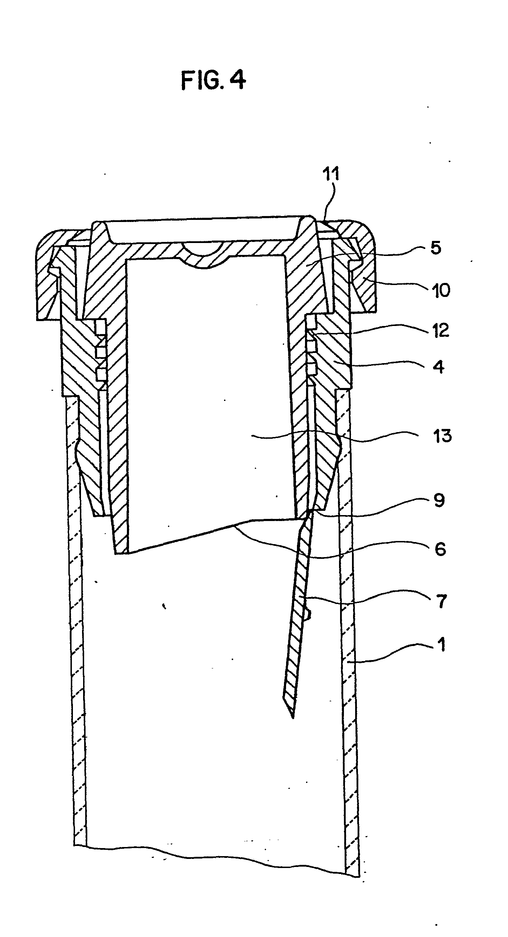

Fig. 4 zeigt den Stopfen nach erfolgtem Druck auf den inneren becherförmigen Teil 5. Der Innenraum des Stopfens 13 ist nun mit dem Innern des Röhrchens verbunden, sodass das Testblut zur Auflösung des Inhaltes durch entsprechende Bewegung vollständig in den Stopfen eindringen kann und das gegebenenfalls im Stopfen festklebende Thromboplastin und Calciumsalz herauslösen kann. Es ist ersichtlich, dass der Rand 6 des inneren becherförmigen Teils 5 des Stopfens den Bodenteil 7 entlang der Reisslinie praktisch vollständig vom äusseren becherförmigen Teil 4 abgetrennt hat. Zur guten Beurteilung des Testergebnises ist es von Vorteil, wenn der Bodenteil 7 nicht vollständig vom äusseren becherförmigen Teil 4 abgetrennt wird. Aus der Zeichnung ist ersichtlich, dass er an einer Stelle 9 mit dem Stopfen verbunden bleibt. Der hineingedrückte Teil 5 bewirkt zusätzlich, dass der Teil 7 nach aussen gedrückt wird, wobei der Innenraum des Stopfens einwandfrei zugänglich ist. In der Darstellung sind ebenfalls die durchgerissenen Sollreiss-Stellen 11 des Sicherungsrings 10 ersichtlich.

Im nachstehenden Beispiel ist die Zubereitung der im Röhrchen enthaltenen Reagentien und ihre Abfüllung näher erläutert.

**Beispiel**

1. Einfüllen des Komplexbildners EDTA in das Röhrchen:

20,2 g $K_2$-EDTA (Ethylendiamin-tetraessissäure-dicaliumsalzdihydrat, M,404,47) werden in einem Messkolben zu 2 l in demineralisiertem Wasser gelöst und von dieser Lösung werden je 1,00 ml in die graduierten Röhrchen abgefüllt. Von der in die Röhrchen eingefüllten Lösung wird das Wasser durch Gefriertrocknung entfernt. Dadurch wird vermieden, dass sich grosse Kristalle bilden, welche leicht aus dem Röhrchen fallen könnten.

Zur Erhöhung der Löslichkeit des $K_2$-EDTA kann ihre Oberfläche erhöht werden, indem der $K_2$EDTA-Lösung 2-5 % Lactose oder Mannit zugesetzt werden.

2. Herstellung einer Calcium-haltigen Thromboplastinsuspension und ihre Abfüllung in den Stopfen:

a) Herstellung der Calciumlösung

59,9 g Calciumgluconat ($C_{12}H_{22}CaO_{14}$ . $H_2O$, M,448,4) werden in einem Becherglas in zirka 1800 ml demineralisiertem Wasser unter Erwärmung aufgelöst. Die Lösung wird abgekühlt und in einen 2 l Messkolben übergeführt, der bis auf 2 l mit demineralisiertem Wasser aufgefüllt wird.

b) Herstellung des Thromboplastins

Ein frisches, auf Eis gekühltes Rinderhirn wird mit 10 g Eis versetzt und mit einem Mixer während 2 min homogenisiert. Das Homogenisat wird 3 × mit 500 ml Aceton extrahiert, wobei das Aceton durch Zentrifugieren abgetrennt wird. Der so gereinigte Rückstand wird auf einer Glasfilternutsche und anschliessend in einem Exzikator getrocknet und danach als Thromboplastin bezeichnet.

c) Herstellung der Calcium-haltigen Thromboplastinsuspension

6 g des unter b) hergestellten Thromboplastins werden mit 400 ml demineralisiertem Wasser gemischt und während 90 s unter Eiskühlung homogenisiert.

Mit einer Vollpipette werden 100 ml gemäss a) hergestellte Calciumgluconatlösung vorgelegt und mit der Thromboplastinsuspension auf 500 g aufgefüllt. Unter Rühren wird je 1,00 g der eisgekühlten Suspension in die zum Röhrchen gehörenden Stopfen eingefüllt. Vor dem Schliessen des Stopfens wird die abgefüllte Suspension sofort eingefroren und gefriergetrocknet, womit die Stabilität des Thromboplastins gewährleistet wird. Anschliessend wird der Stopfen, welcher nun 13,3 µmol Calcium und 12 mg Thromboplastin enthält, geschlossen und auf das mit EDTAbeschichtete Röhrchen aufgesetzt.

**Patentansprüche**

1. Veterinärmedizinischer Reagentiensatz in Teströhrchen und dazugehörendem Stopfen zur Bestimmung des Calciumgehaltes im Blut, indem eine abgemessene Menge Blut mit einer vorbestimmten Menge Komplexbildner und anschliessend einer vorbestimmten Menge Calciumionen versetzt wird, so dass entsprechend dem Calciumgehalt des Testblutes eine vollständige, teilweise oder keine Koagulation stattfindet, dadurch gekennzeichnet, dass der Komplexbildner im Teströhrchen (1), welches zur Blutaufnahme dient, vorliegt und vorbestimmte Mengen eines Calciumsalzes und ein Koagulationsbeschleuniger in einem Hohlraum des zum Teströhrchen gehörenden Stopfens (2) vorhanden sind, wobei der Stopfen so ausgebildet ist, dass sein Inhalt in das mit dem Stopfen verschlossene Röhrchen freigegeben werden kann.

2. Reagentiensatz in Teströhrchen und dazugehörendem Stopfen gemäss Anspruch 1, dadurch gekennzeichnet, dass der Stopfen aus zwei becherförmigen Teilen (4 und 5) besteht, die unter Bildung eines geschlossenen dichten Hohlraumes zur Aufnahme des Calciumsalzes und des Thromboplastins konzentrisch beweglich zusammengefügt sind, der innere becherförmige Teil (5) oben verschlossen ist und der Rand (6) an der offenen Seite bezüglich des Bodens zur teilweisen Abtrennung des Bodenteils (7) mindestens teilweise abgeschrägt ist und der äussere becherförmige Teil (4), der den Abschluss zum Inneren des Teströhrchens (1) bildet, unten durch den Bodenteil (7) verschlossen ist, so dass, wenn der innere becherförmige Teil, der oben aus dem Stopfen herausragt, in den äusseren becherförmigen Teil (4) hineingedrückt wird, der Bodenteil (7) des äusseren becherförmigen Teils (4) deckelartig geöffnet wird, und der Bodenteil (7) vorzugsweise noch an einer Stelle (9) festgehalten wird, wobei der Inhalt des Stopfens dem Testblut im Inneren des Röhrchens (1) zugänglich gemacht wird.

3. Reagentiensatz in Teströhrchen und dazugehörendem Stopfen gemäss Anspruch 2, dadurch gekennzeichnet, dass der innere Teil (5) des Stopfens (2) zur Sicherung gegen ungewolltes Freisetzen des Inhalts des Stopfens einen Sicherungsring (10) aufweist, mit dem er durch mehrere Sollreiss-Stellen (11) verbunden ist.

4. Reagentiensatz in Teströhrchen und dazugehörendem Stopfen nach Anspruch 2 oder 3, dadurch gekennzeichnet, dass der Bodenteil (7) des Stopfens, welcher den inneren Abschluss zum Röhrchen (1) bildet, am Rand eine Reisslinie (8) aufweist.

5. Reagentiensatz in Teströhrchen und dazugehörendem Stopfen nach einem der Ansprüche 1-4, dadurch gekennzeichnet, dass das Röhrchen (1) eine Kalibrierung (3) aufweist, die den der Blutmenge entsprechenden Schwellenwert des Blutcalciumgehaltes in mg% bzw. mmol/l angibt, wobei sich diese Angaben effektiv auf den Serumcalciumgehalt beziehen.

6. Reagentiensatz in Teströhrchen und dazugehörendem Stopfen, dadurch gekennzeichnet, dass der Komplexbildner im Röhrchen Ethylendiamin-tetraessigsäure, Ethylenglykol-bis-(2-aminoethyl)-tetraessigsäure oder Salze davon, insbesondere das Kalium- oder Natriumsalz ist.

7. Reagentiensatz in Teströhrchen und dazugehörendem Stopfen gemäss einem der Ansprüche 1-6, dadurch gekennzeichnet, dass das Calciumsalz im Stopfen Calciumchlorid, Calciumascorbat, Calciumboroglukonat, Calciumbromid, Calciumbromolactobionat, Calciumcarbonat, Calciumcyclamat, Calcium-2-ethylbutanoat, Calciumfluorid, Calciumformiat, Calciumgluconat, Calciumglycerophosphat, Calciumhypophosphit, Calciumiodid, Calciumlactat, Calciumlaevulinat, Calciummethionat, Calciumnitrat, Calciumnitrit, Calciumpantothenat, Calciumpropionat, Calciumselenat, Calciumsuccinat, Calciumsulfat, Calciumsulfit, Calciumtartrat, Calciumthioglycollat oder Calciumthiosulfat ist.

8. Reagentiensatz in Teströhrchen und dazugehörendem Stopfen nach einem der Ansrüche 1-7, dadurch gekennzeichnet, dass der Koagulationsbeschleuniger Thromboplastin ist.

9. Reagentiensatz in Teströhrchen und dazugehörendem Stopfen nach Anspruch 8, dadurch gekennzeichnet, dass das Thromboplastin aus tierischem Hirngewebe, vorzugsweise vom Pferd, Rind, Schwein, Schaf, Kaninchen oder von der Ziege stammt.

10. Verfahren zur Herstellung des Reagentiensatzes in Teströhrchen und dazugehörendem Stopfen nach einem der Ansprüche 8 oder 9, dadurch gekennzeichnet, dass

A) eine Lösung definierter Konzentration des Komplexbildners in demineralisiertem Wasser hergestellt wird, ein definiertes Volumen dieser Lösung in das Teströhrchen gegeben wird und das Wasser durch Gefriertrocknung entfernt wird;

B) Thromboplastin aus frischen tierischen Zellen extrahiert wird, das erhaltene Thromboplastin zusammen mit einer Calciumsalzlösung in eine Suspension mit vorbestimmter Konzentration gebracht wird, eine vorbestimmte Menge dieser Suspension in den Stopfen des Teströhrchens gebracht wird, die abgefüllte Suspension eingefroren und gefriergetrocknet und der Stopfen mit der getrockneten Lösung verschlossen und auf das den Komplexbildner enthaltende kalibrierte Röhrchen aufgesetzt.wird.

11. Verfahren zur semiquantitativen Bestimmung des Calciumgehaltes im Blut, dadurch gekennzeichnet, dass der Reagentiensatz im Teströhrchen und dazugehörendem Stopfen gemäss einem der Ansprüche 1-8 verwendet wird, indem das zu testende Blut in vorbestimmter Menge in das Teströhrchen gebracht wird, das Röhrchen durch den Stopfen wieder verschlossen wird, das Blut oder Plasma mit dem Komplexbildner durch mässiges Bewegen vermischt wird, nach vollständigem Lösen des Komplexbildners der Koagulationsbeschleuniger und das Calciumsalz im Stopfen der Testlösung zugänglich gemacht wird und der Inhalt des Röhrchens mit dem Inhalt des Stopfens vollständig vermischt, mindestens 5 min gewartet und der Test beurteilt wird, wobei eine Koagulation zeigt, dass der Blutcalciumgehalt über dem gewählten Schwellenwert liegt, und wenn keine Koagulation eintritt, der Blutcalciumgehalt unter dem Schwellenwert liegt.

**Revendications**

1. Ensemble médico-vétérinaire de réactifs dans un tube à essais et dans le bouchon de ce dernier pour déterminer le taux de calcium dans le sang, par lequel on mélange une quantité mesurée de sang avec une quantité prédéterminée de complexant et ensuite une quantité prédéterminée de ions de calcium afin que, suivant le taux de calcium du sang en test, il se produise une coagulation complète, partielle ou aucune coagulation, caractérisé en ce que le complexant se trouve dans le tube à essais (1) servant à recevoir le sang, et que des quantités prédéterminées d'un sel de calcium et un coagulant se trouvent dans un espace vide du bouchon (2) faisant partie du tube à essais, ce bouchon étant construit de manière telle que son contenu puisse passer librement dans le tube qui est fermé par le bouchon.

2. Ensemble médico-vétérinaire de réactifs dans un tube à essais et dans le bouchon de ce dernier selon la revendication 1, caractérisé en ce que le bouchon se compose de deux parties en forme de coupe (4 et 5) qui pour former un espace vide hermétiquement fermé destiné à recevoir le sel de calcium et la thromboplastine sont assemblées concentriquement de façon mobile, en ce que la partie intérieure en forme de coupe (5) est fermée par en haut et que le bord (6) du côté qui s'ouvre par rapport au fond pour séparer partiellement le fond (7), est au moins partiellement oblique et en ce que la partie extérieure en forme de coupe (4) qui forme l'obturation de l'intérieur du tube à essais (1) est fermée par en bas par le fond (7) de sorte que lorsque la partie intérieure en forme de coupe qui ressort au haut du bouchon est repoussée dans la partie extérieure en forme de coupe (4), la partie de fond (7) de la partie extérieure en forme de coupe (4) s'ouvre comme un couvercle et reste de préférence fixée à un endroit (9), permettant ainsi au contenu du bouchon d'accéder librement au sang à tester à l'intérieur du tube (1).

3. Ensemble médico-vétérinaire de réactifs dans un tube à essais et dans le bouchon de ce dernier selon la revendication 2, caractérisé en ce que la partie intérieure (5) du bouchon (2) présente un anneau de sécurité (10) pour empêcher un épanchement non désiré du contenu du bouchon, qui lui est lié par plusieurs renforcement (11).

4. Ensemble médico-vétérinaire de réactifs dans un tube à essais et dans le bouchon de ce dernier selon l'une des revendications 1-4, caractérisé en ce que le fond du bouchon, qui forme l'obturation intérieure du bouchon, présente à son bord une ligne de déchirure.

5. Ensemble médico-vétérinaire de réactifs dans un tube à essais et dans le bouchon de ce dernier selon l'une des revendications 1 à 4, caractérisé en ce que le tube (1) présente une graduation (3) qui indique en mg, respect. en mmol/l la valeur de seuil du taux de calcium dans le sang correspondant à la quantité de sang, ces indications ayant trait effectivement au taux de calcium dans le sérum.

6. Ensemble médico-vétérinaire de réactifs dans un tube à essais et dans le bouchon de ce dernier caractérisé en ce que l'agent complexant dans le tube est l'acide éthylènediamine-tétraacétique, l'acide éthylèneglycol-bis-(2-aminoéthyltétraacétique ou des sels de ceux-ci, en particulier sel de potassium ou sel de sodium.

7. Ensemble médico-vétérinaire de réactifs dans un tube à essais et dans le bouchon de ce dernier selon l'une des revendications 1 à 6, caractérisé en ce que le sel de calcium dans le bouchon est le chlorure de calcium, l'ascorbate de calcium, le borogluconate de calcium, le bromure de calcium, le bromolactobionate de calcium, le carbonate de calcium, le cyclamate de calcium, le 2-éthylbutanoate de calcium, le fluorure de calcium, le formiate de calcium, le gluconate de calcium, le glycérophosphate de calcium, l'hypophosphite du calcium, le iodure de calcium, le lactate de calcium, le laevulinate de calcium, le méthionate de calcium, le nitrate de calcium, le nitrite de calcium, le pantothénate de calcium, le propionate de calcium, le sélénate de calcium, le succinate de calcium, le sulfate de calcium, le sulfite de calcium, le tartrate de calcium, le thioglycollate de calcium ou le thiosulfate de calcium.

8. Ensemble médico-vétérinaire de réactifs dans un tube à essais et dans le bouchon de ce dernier selon l'une des revendications 1 à 7, caractérisé en ce que le coagulant est la thromboplatine.

9. Ensemble de réactifs dans un tube à essais et dans le bouchon de ce dernier selon la revendication 8, caractérisé en ce que la thromboplastine issue de tissu de cerveaux animaux provient de préférence du cheval, du boeuf, du porc, du mouton, du lapin ou de la chèvre.

10. Procédé de préparation de l'ensemble de réactifs dans un tube à essais et dans le bouchon de ce dernier selon l'une des revendications 8 ou 9, caractérisé en ce que

(A) on prépare dans de l'eau déminéralisée une solution du complexant dans une concentration définie, on met un volume défini de cette solution dans le tube à essais et on enlève l'eau par lyophilisation;

(B) on extrait la thromboplastine de cellules animales fraiches, on met en suspension dans une concentration prédéterminée la thromboplastine obtenue ainsi qu'une solution de calcium, on met une quantité prédéterminée de cette suspension dans le bouchon du tube à essais, on gèle et lyophilise la dite quantité de suspension et on ferme le bouchon renfermant la solution sèche et on le place sur le tube gradué qui contient le complexant.

11. Procédé pour déterminer le taux de calcium dans le sang de manière semiquantitative, caractérisé en ce qu'on utilise l'ensemble de réactifs en tube à essais accompagné de son bouchon selon l'une des revendications 1 à 8, selon lequel on met une quantité prédéterminée du sang à tester dans le tube à essais, on ferme à nouveau le tube au moyen du bouchon, on mélange le sang ou le plasma avec le complexant sous agitation moyenne, après dissolution complète du complexant, on assure le libre passage du coagulant et du sel de calcium qui sont dans le bouchon vers la solution à tester dans le tube et on mélange intimement le contenu du tube avec celui du bouchon, après au moins 5 minutes d'attente, on évalue le résultat du test, la présence d'une coagulation indiquant que le taux de calcium dans le sang est au dessus de la valeur de seuil choisie et l'absence de coagulation indiquant que le taux de calcium dans le sang est en dessous de ce seuil.

## Claims

1. Veterinary medical reagent kit in test tube and stopper belonging to it to determine the calcium content in the blood in that a measured amount of blood is mixed with a predetermined amount of complexing agent and then a predetermined amount of calcium ions so that complete, partial or no coagulation takes place, depending upon the calcium content of the test blood, characterized in that there is the complexing agent in the test tube (1), which serves to receive the blood, and there are predetermined amounts of a calcium salt and a coagulation catalyst in a cavity of the stopper (2) belonging to the test tube, the stopper being formed in such a way that its content can be released into the test tube closed off by the stopper.

2. Reagent kit in test tube and stopper belonging to it according to claim 1, characterized in that the stopper consists of two cup-shaped parts (4 and 5), which are fitted together in a concentrically movable way forming a closed, tight cavity to receive the calcium salt and the thromboplastin, the inner cup-shaped part (5) is closed at the top, and the edge (6) is at least partially bevelled on the open side in relation to the bottom for partial separation of the bottom part (7), and the outer cup-shaped part (4), which forms the closure on the interior of the test tube (1), is closed below by the bottom part (7) so that when the inner cup-shaped part, which protrudes above out of the stopper, is pressed into the outer cup-shaped part (4), the bottom part (7) of the outer cup-shaped part (4) is opened like a cap, and the bottom part (7) is still held tightly, preferably at one place (9), the contents of the stopper being made accessible to the test blood inside of the test tube (1).

3. Reagent kit in test tube and stopper belonging to it according to claim 2, characterized in that, to secure against unwanted release of the contents of the stopper, the inner part (5) of the stopper (2) has a safety ring (10) with which it is connected through several predetermined tearing points (11).

4. Reagent kit in test tube and stopper belonging to it according to claim 2 or 3, characterized in that the bottom part (7) of the stopper, which forms the interior closure to the test tube (1), has a tear line (8) on the edge.

5. Reagent kit in test tube and stopper belonging to it according to one of the claims 1-4, characterized in that the test tube (1) has a calibration (3), which indicates the threshold value of the calcium content of the blood in mg % or m/moll, corresponding to the quantity of blood, these data actually referring to the serum calcium content.

6. Reagent kit in test tube and stopper belonging to it, characterized in that the complexing agent in the test tube is ethylenediamine-tetraacetic acid, ethylene-glycol-bis-(2-aminoethyl)-tetraacetic acid or salts, of these particularly potassium salt or sodium salt.

7. Reagent kit in test tube and stopper belonging to it according to one of the claims 1-6, characterized in that the calcium salt in the stopper is calcium chloride, calcium ascorbate, calcium borogluconate, calcium bromide, calcium bromolactobionate, calcium carbonate, calcium cyclamate, calcium 2-ethylbutanoate, calcium fluoride, calcium formate, calcium gluconate, calcium glycerophosphate, calcium hypophosphite, calcium iodide, calcium lactate, calcium levulinate, calcium methionate, calcium nitrate, calcium nitrite, calcium pantothenate, calcium propionate, calcium selenate, calcium succinate, calcium sulfate, calcium sulfite, calcium tartrate, calcium thioglycollate or calcium thiosulfate.

8. Reagent kit in test tube and stopper belonging to it according to one of the claims 1-7, characterized in that the coagulation catalyst is thromboplastin.

9. Reagent kit in test tube and stopper belonging to it according to claim 8, characterized in that the thromboplastin comes from animal cerebral tissue, preferably horse, cattle, pig, sheep, rabbit or goat.

10. Process for making the reagent kit in test tube and the stopper belonging to it according to one of the claims 8 or 9, characterized in that

A) a solution of defined concentration of the complexing agent is produced in demineralized water, a defined volume of this solution is put into the test tube and the water removed by freeze-drying;

B) thromboplastin is extracted from fresh animal cells, the thromboplastin obtained is put into a suspension of predetermined concentration with a calcium salt solution, a predetermined quantity of this suspension is put into the stopper of the test tube, the filled suspension is frozen and freeze-dried and the stopper with the dried solution closed and put on the calibrated test tube containing the complexing agent.

11. Process for semiquantitative determination of the calcium content in the blood, characterized in that the reagent kit in the test tube and the stopper belonging to it according to one of the claims 1-8 is used, in that the blood to be tested is put into the test tube in a predetermined quantity, the tube is closed again with the stopper, the blood or plasma is mixed with the complexing agent through moderate motion, the coagulation catalyst and the calcium salt in the stopper is made accessible to the test solution after complete dissolving of the complexing agent, and the content of the tube is completely mixed with the content of the stopper, one waits for at least 5 minutes and evaluates the test, coagulation showing that the calcium content of the blood lies above the selected threshold value, and if no coagulation takes place, the calcium content of the blood lies under the threshold value.

# FIG. 1

## FIG.2

## FIG.3

# FIG. 4